# EUROPEAN PATENT APPLICATION

(11) **EP 3 677 270 A1**
(43) Date of publication of application: **08.07.2020**
(21) Application number: 18852611.5
(22) Date of filing: 09.08.2018
(51) Int. Cl.: A61K 33/04, A61K 47/59, A61K 47/04, A61K 41/00, A61P 35/00

(54) **SELENIUM-DOPED BLACK PHOSPHORUS PRODRUG AND PREPARATION METHOD THEREFOR**

(30) Priority: 29.08.2017 CN 201710754749
(71) Applicant: Shenzhen University, Shenzhen, Guangdong 518055 (CN)
(72) Inventor: ZHANG, Han, Shenzhen Guangdong 518055 (CN); QIU, Meng, Shenzhen Guangdong 518055 (CN)
(74) Representative: Mewburn Ellis LLP
(86) International application number: PCT/CN2018/099567
(87) International publication number: WO 2019/042108

(57) **Abstract**

Disclosed is a selenium-doped black phosphorus prodrug comprising selenium-doped black phosphorus nanosheets and polyethylene glycol amine coating the surface of the selenium-doped black phosphorus nanosheets. The selenium-doped black phosphorus nanosheets comprise black phosphorus nanosheets and selenium elements doped in the black phosphorus nanosheets, with the selenium elements replacing positions of a portion of the phosphorus atoms in the black phosphorus crystal lattice. The selenium-doped black phosphorus prodrug is a controlled-release prodrug of selenium elements, which can realize the near-infrared light controlled-release of selenium elements, thereby controllably regulating selenium content in the human body, regulating human immunity, and preventing and treating cancers. Also provided is a method for preparing the selenium-doped black phosphorus prodrug.

## Description

### CROSS-REFERENCE TO RELATED APPLICATIONS

The present application claims the priority of a Chinese patent application No. 2017107547499, which was filed on August 29, 2017, titled "Selenium-Doped Black Phosphorus Prodrug and Preparation Method Therefor", the entirety of which is herein incorporated by references.

### FIELD OF THE INVENTION

The invention relates to nanomaterial for biomedicines, and more particularly to selenium-doped black phosphorus prodrug and preparation method therefor.

### BACKGROUND OF THE INVENTION

Cancer seriously harms human life and health. Statistics show that cancer causes more deaths, compared with AIDS, malaria or tuberculosis. One in five deaths are caused by cancer. Breast cancer is the most common malignancy in women, with a mortality rate of 17% among all women's cancer. At present, regardless of chemotherapy, surgery or radiation therapy for breast cancer, it will have great side effects on the human body. If malignant breast tumor cells metastasize, it is difficult to completely cure them through the above-mentioned treatment. Although a lot of human, material and financial resources have been invested in cancer research, progress has been limited, effective treatment remains a huge challenge for humans.

Some studies show that a trace amount of selenium is closely related to human health. Studies at home and abroad have proven that selenium (Se), an essential trace element of the human body, has important physiological functions and a wide range of pharmacological effects. Either the deficiency or excess of selenium is closely related to human life and health. A large number of statistical data and clinical studies at home and abroad have proven that selenium deficiency can cause dysfunction of important organs, leading to the occurrence of many serious diseases. In many areas, people control these diseases through selenium supplementation. Numerous pharmacological and clinical studies have further proven that selenium is irreplaceable due to its physiological functions in human body. Selenium is a regulator of cancer gene expression. Many studies have shown that selenium can inhibit the biosynthesis of proteins and DNA and delay the mitotic phase in the cell cycle. At the same time, it can prolong the quiescent period before cell mitosis and is good for DNA repair. Selenium can reduce the malignant transformation of cells by inhibiting or regulating the hyperplasia stimulated by proliferation and have a two-way regulating effect on promoting the differentiation and inhibiting the division of tumor cells. In the process of chemical carcinogenesis, selenium can inhibit many changes caused by anticarcinogens at the initiation and development stages. Therefore, selenium is considered to be a regulator of cancer gene expression.

Although the inorganic compounds of selenium have high anti-cancer efficacy, some experiments have proven that the inorganic compounds of selenium have cumulative toxicity and mutagenic effects, and the dosage is difficult to be controlled. In general, inorganic compounds of selenium (such as selenate and selenite) are known to cause genotoxic effects and are not suitable for medical use, especially at high doses, and are therefore difficult to use in clinical applications.

### SUMMARY OF THE INVENTION

In view of this, the present invention provides a selenium-doped black phosphorus prodrug for a controlled-release prodrug of selenium. This prodrug can achieve NIR-responsive controlled release of selenium so as to regulate the selenium content in the human body, regulate human immunity and prevent and treat cancer without toxic side effects.

Specifically, in a first aspect, the present invention provides a selenium-doped black phosphorus prodrug comprising selenium-doped black phosphorus nanosheets and polyethylene glycol amine coated on the surface of the selenium-doped black phosphorus nanosheets, wherein the selenium-doped black phosphorus nanosheets comprises black phosphorus nanosheets and selenium doped in the black phosphorus nanosheets, and wherein selenium replaces a part of phosphorus atoms in the black phosphorus crystal lattice.

In the present invention, selenium is doped in the black phosphorus nanosheets to achieve NIR-responsive controlled release of selenium. Meanwhile, the black phosphorus nanosheets is coated with polyethylene glycol amine, making it have good biocompatibility. As a result, it can be excreted through biodegradation or normal physiological pathways without toxic side effects on the organism, along with biological safety. The present invention provides a new way for the application of selenium in the field of biomedicine.

The selenium is doped in the selenium-doped black phosphorus nanosheets at a mass concentration of 0.1-5%. Alternatively, selenium is included at a mass concentration of 1-4%, further preferably 2-3%. A suitable selenium content would meet the requirements of the prodrug for the selenium content and is capable of securing a good optical absorption performance, photo-thermal performance of black phosphorus nanosheets, thus realizing the effective release of selenium.

The length and width dimensions of the selenium-doped black phosphorus nanosheets are in a range of 50nm-200nm. The thickness of the selenium-doped black phosphorus nanosheets is in a range of 1 nm-5nm. Alternatively, dimensions of the selenium-doped black phosphorus nanosheets are in a range of 50nm-150nm, 100nm-150nm, 120nm-180nm, 160nm-200nm. Alternatively, the thickness is in a range of 1-3 nm or 2-4 nm. A suitable size of black phosphorus nanosheets would effectively improve the passive targeting of cancer cells of the black phosphorus nanosheets. A suitable thickness would provide a suitable forbidden band width, thereby more effectively absorbing near-infrared light and converting it into thermal energy.

The mass ratio of the selenium-doped black phosphorus nanosheets to polyethylene glycol amine is 1: 0.2-10. Alternatively, the mass ratio is in a range of 1: 1-8, or 1: 2-6, or 1: 3-5. Modification using a suitable amount of polyethylene glycol amine would improve the biocompatibility and stability of black phosphorus nanosheets.

In the present invention, the polyethylene glycol amine comprises at least one compound selected from the group consisting of methyl polyethylene glycol amine (CH₃-PEG-NH₂), methoxy polyethylene glycol amine (CH₃O-PEG-NH₂, abbreviated as mPEG-NH₂), and polyethylene glycol diamine (NH₂-PEG-NH₂). The polyethylene glycol amine is adsorbed on the surface of the selenium-doped black phosphorus nanosheets due to electrostatic force, and the weight average molecular weight of the polyethylene glycol amine is in a range of 2000-30000. Polyethylene glycol amine can effectively prevent the aggregation of black phosphorus nanosheets. In addition, polyethylene glycol amine has both hydrophilic properties and polarity, which can significantly inhibit the recognition of the immune system, reduce the phagocytosis of the monocyte-phagocytic system, and effectively prolonging the blood circulation in nanosheets, thus improving the bioavailability of drugs and the enrichment of cancer sites.

In the present invention, the black phosphorus nanosheets have a very high ratio of surface area to mass. The black phosphorus nanosheets, on the one hand, can absorb near-infrared irradiation to provide excellent photo-thermal performance; on the other hand, the surface of the black phosphorus nanosheets is full of negative charges, which improve the electrostatic adsorption with polyethylene glycol amine.

The selenium-doped black phosphorus prodrug provided by the first aspect of the present invention is NIR-responsive and enables controlled release of selenium, thus achieving effective control of the selenium content of the human body, and control of the growth of tumor cells at the lesion site. Further, this prodrug is controlled degradable and has extremely high clinical value for improving human immunity and preventing and treating cancer.

In a second aspect, the present invention provides a method for preparing a selenium-doped black phosphorus prodrug, comprising:
sealing red phosphorus, tin, tin tetraiodide and selenium in a silicon glass vacuum tube at a mass ratio of (200-500): (10-20): (5-10): (0.5-10);
placing the silicon glass vacuum tube horizontally in a heating furnace, followed by heating the silicon glass vacuum to 700-800°C and maintaining for 1-5 hours, and then cooling to 450-550 °C and maintaining for 5-9 hours, and then further cooling to 100-200 °C and maintaining for 6-10 hours, followed by cooling to room temperature to obtain selenium-doped black phosphorus crystals in the silicon glass vacuum tube;
dispersing the selenium-doped black phosphorus crystals into an aqueous phase by liquid exfoliation to obtain suspended selenium-doped black phosphorus nanosheets;
coating the selenium-doped black phosphorus nanosheets with polyethylene glycol amine while ultrasonicating and stirring to obtain a selenium-doped black phosphorus prodrug, which comprises:
   selenium-doped black phosphorus nanosheets, and
   polyethylene glycol amine coated on the surface of the selenium-doped black phosphorus nanosheets,
   wherein the selenium-doped black phosphorus nanosheets comprises black phosphorus nanosheets and selenium doped in the black phosphorus nanosheets, and
   wherein the selenium replaces a part of phosphorus atoms in the black phosphorus crystal lattice.

In the present invention, alternatively, the step of heating is performed at a heating rate of 1-5°C/min, preferably 2-4°C/min, and 1.5-3°C/min. By heating to 700-800°C and maintaining for 1-5 hours, the raw materials in the silicon glass tube would completely sublimate and react. After that, it is cooled to 450-550 °C and maintained for 5-9 hours, and then further cooled to 100-200 °C and maintained for 6-10 hours, and then cooled to room temperature. The sublimated gas is cooled and crystallized on the inner wall of the silicon glass tube to form selenium-doped black phosphorus crystals in such a gradient cooling process. The step of cooling to 450-550 °C is performed at a cooling rate of 1-3°C/min, and the step of cooling to 100-200 °C is performed at a cooling rate of 1-3°C/min. The cooling process at a suitable rate is beneficial to the formation of black phosphorus crystals and uniform doping of selenium in the black phosphorus crystals.

In the present invention, mass of the selenium is 0.1-5% of mass of the red phosphorus. Further, mass of the selenium may be 1-4% or 2-3% of mass of the red phosphorus.

In the present invention, mass of the tin is 2-10% of mass of the red phosphorus, preferably 3% -8%, 4% -6%, 5% -7%. The mass of the tin tetraiodide is 1-5% of the mass of the red phosphorus. For example, 1%, 2%, 3%, 4%, or 5%.

Alternatively, the length of the silicon glass tube is 200 mm and the diameter is 10 mm.

The liquid exfoliation process employed in the present invention can be any method for exfoliating black phosphorous into black phosphorous sheets in the art, and is not limited. Adjusting operating parameters of the liquid exfoliation process would provide selenium-doped black phosphorus nanosheets of different dimensions.

Alternatively, the ultrasonic treatment uses an ultrasonic frequency of 3000-4500HZ and lasts for 0.5-2 hours. The step of stirring is performed at a speed of 800rpm-1200rpm for 2-4 hours. The ultrasonic treatment and stirring may be performed sequentially, for example, the ultrasonic treatment may be performed for 0.5 hours, before stirring for 3 hours.

Alternatively, the length and width dimensions of the selenium-doped black phosphorus nanosheets are in a range of 50nm-200nm; the thickness of the selenium-doped black phosphorus nanosheets is in a range of 1 nm-5nm. Alternatively, dimensions of the selenium-doped black phosphorus nanosheets are in a range of 50nm-150nm, 100nm-150nm, 120nm-180nm, 160nm-200nm. Alternatively, the thickness is in a range of 1-3 nm or 2-4 nm.

The mass ratio of the selenium-doped black phosphorus nanosheets to polyethylene glycol amine is in a range of 1: 0.2-10. Alternatively, the mass ratio is in a range of 1: 1-8, or 1: 2-6, or 1: 3-5. In the present invention, the polyethylene glycol amine comprises at least one compound selected from the group consisting of methyl polyethylene glycol amine (CH₃-PEG-NH₂), methoxy polyethylene glycol amine (CH₃O-PEG-NH₂, abbreviated as mPEG-NH₂) and polyethylene glycol diamine (NH₂-PEG-NH₂). The polyethylene glycol amine is adsorbed on the surface of the selenium-doped black phosphorus nanosheets due to electrostatic force, and the weight average molecular weight of the polyethylene glycol amine is in a range of 2000-30000.

The method for preparing the selenium-doped black phosphorus prodrug provided by the second aspect of the present invention is simple and easy to implement, and can realize industrial production.

### BRIEF DESCRIPTION OF THE DRAWINGS

Fig. 1 shows structure of a selenium-doped black phosphorus prodrug prepared in Example 1 of the present invention.
Fig. 2 shows a photo of selenium-doped black phosphorus crystals prepared at step (2) of Example 1 of the present invention.
Fig. 3 shows a Raman spectrum of selenium-doped black phosphorus crystals prepared at step (2) of Example 1 of the present invention.
Fig. 4 shows an ultraviolet-visible absorption spectrum of the selenium-doped black phosphorus crystals prepared at step (2) of Example 1 of the present invention.
Fig. 5 shows a transmission electron microscope (SEM) image of a selenium-doped black phosphorus prodrug prepared in Example 1 of the present invention.
Fig. 6 shows release and degradation of a selenium-doped black phosphorus prodrug prepared according to one embodiment of the present invention.
Fig. 7 shows a transmission electron microscope (SEM) image of a selenium-doped black phosphorus prodrug prepared in Example 2 of the present invention.

### DETAILED DESCRIPTION OF THE INVENTION

The following describes preferred embodiments of the present invention. It should be noted that those skilled in the art can make several improvements or modifications without departing from the principles of the embodiments of the present invention. These improvements or modification are also considered as the protection scope of the present invention.

### Example 1

A method for preparing selenium-doped black phosphorus prodrug comprises the following steps.
(1) 500 mg of red phosphorus, 20 mg of tin, 10 mg of tin tetraiodide and 0.5 mg of selenium were sealed in a silicon glass vacuum tube.
(2) The silicon glass tube was placed horizontally in a heating furnace and heated at a heating rate of 3°C/min to 750°C and maintained for 1 hour, and then cooled at a cooling rate of 3°C/min to 500°C and maintained for 7.5 hours. Further, the glass tube was cooled at a cooling rate of 3°C/min to 150 °C and maintained for 8 hours, and then cooled to room temperature to obtain selenium-doped black phosphorus crystals deposited on the end of the silicon glass tube.
(3) The selenium-doped black phosphorus crystals were dispersed in the aqueous phase by liquid exfoliation to obtain suspended selenium-doped black phosphorus nanosheets, which were then coated with polyethylene glycol amine sequentially by ultrasonicating and stirring so as to obtain the selenium-doped black phosphorus prodrug, wherein the mass ratio of selenium-doped black phosphorus nanosheets to polyethylene glycol amine was 1:2. The ultrasonic treatment was performed at a frequency of 4000HZ for 2 hours, and the magnetic stirring was performed at a speed of 800 rpm for 4 hours.

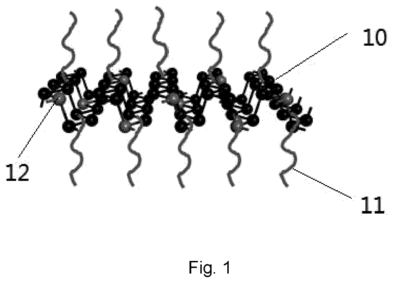

Fig. 1 shows structure of a selenium-doped black phosphorus prodrug prepared in Example 1 of the present invention. The prodrug comprises selenium-doped black phosphorus nanosheets 10 and polyethylene glycol amine 11 coated on the surface of the selenium-doped black phosphorus nanosheets, wherein the selenium-doped black phosphorus nanosheets 10 comprises black phosphorus nanosheets and selenium 12 doped in the black phosphorus nanosheets, and wherein the selenium 12 replaces a part of phosphorus atoms in the black phosphorus crystal lattice.

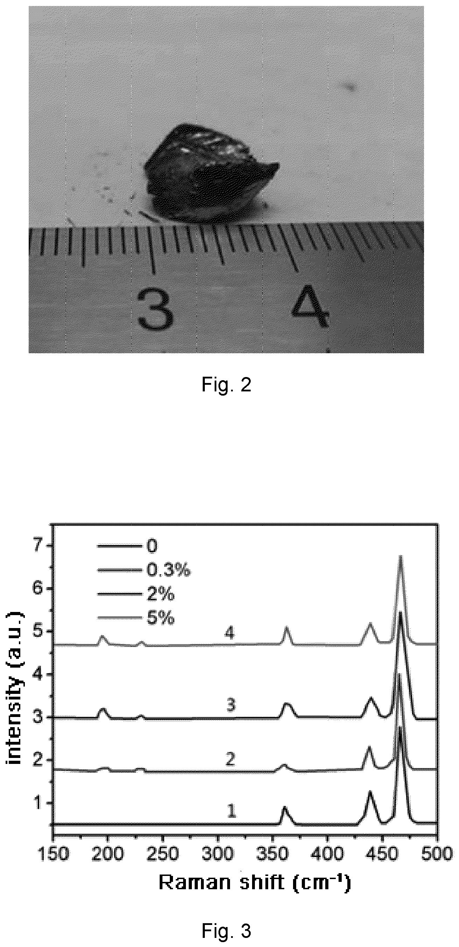

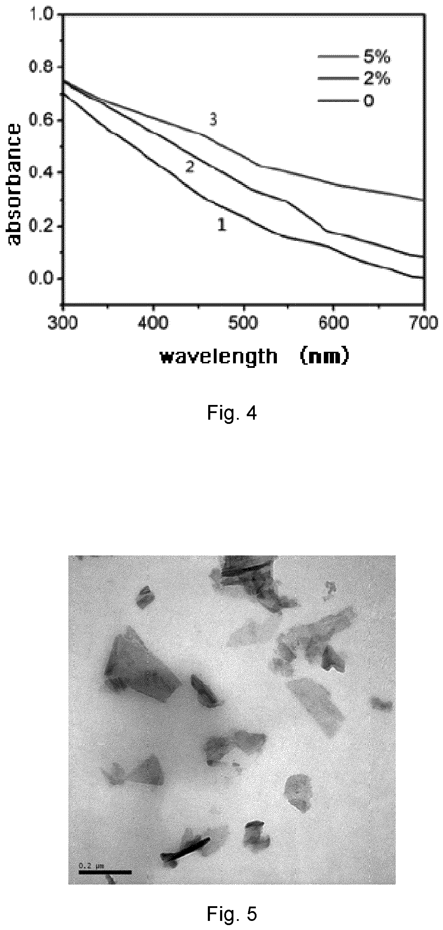

Fig. 2 shows a photo of selenium-doped black phosphorus crystals prepared at step (2) of Example 1 of the present invention. Fig. 3 and Fig. 4 show a Raman spectrum and an ultraviolet-visible absorption spectrum of the selenium-doped black phosphorus crystals prepared at step (2) of Example 1 of the present invention. In Fig. 3, curve 1 is the Raman spectrum of black phosphorus crystals without selenium. Curves 2, 3, and 4 are the Raman spectra of black phosphorus crystals doping with 0.3%, 2%, and 5% of selenium, respectively. It can be seen from Fig. 3 that new Raman peaks of selenium appear in curves 2, 3, and 4, indicating that the selenium is successfully doped. In Fig. 4, curve 1 is an ultraviolet-visible absorption spectrum of black phosphorus crystals without selenium. Curves 2 and 3 are ultraviolet-visible absorption spectrum of black phosphorus crystals doping with 2% and 5% of selenium, respectively. It can be seen from Fig. 4 that the absorption spectrum undergoes a significant red shift with increasing selenium content, thereby improving the near-infrared response of selenium-doped black phosphorus crystals, which is beneficial to the controlled-release drugs for deep tissues.

Fig. 5 shows a transmission electron microscope (SEM) image of a selenium-doped black phosphorus prodrug prepared in Example 1 of the present invention. The size of the selenium-doped black phosphorus prodrug is in a range of 100-200 nm.

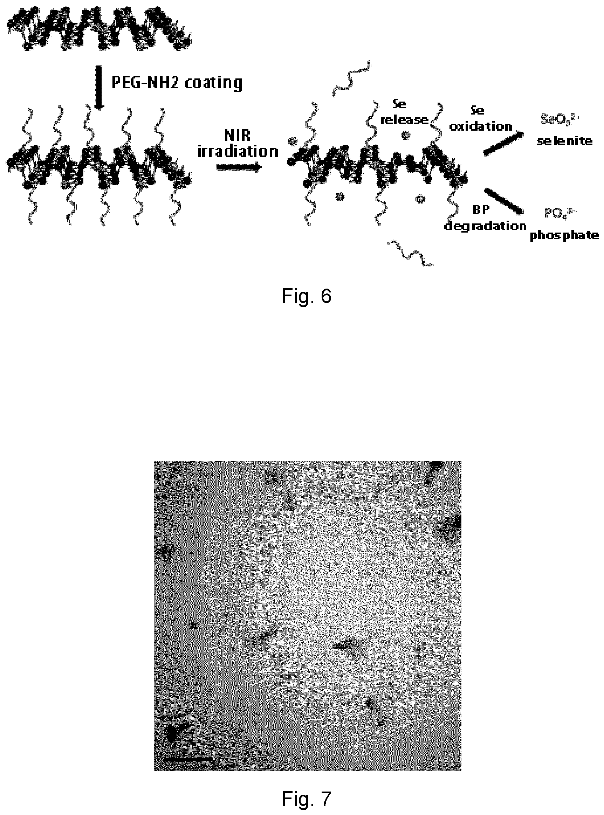

The selenium-doped black phosphorus prodrug prepared according to the embodiment of the present invention under the near-infrared irradiation at 808 nm, on the one hand, would exhibit excellent light-to-heat conversion due to the black phosphorus nanosheets per se, so as to realize photothermal treatment. On the other hand, the selenium-doped black phosphorus nanosheets can be degraded under near-infrared irradiation. During the degradation, phosphorus atoms are oxidized to non-toxic compounds such as phosphoric acid, and selenium atoms are oxidized to selenite ions and release gradually. Selenite has anti-cancer properties and improves immunity, so that the selenium-doped black phosphorus prodrug prepared according to the embodiment of the present invention can effectively inhibit the growth of cancer cells. Since black phosphorus nanosheets have good biocompatibility and can be excreted through biodegradation or normal physiological pathways, the selenium-doped black phosphorus prodrugs prepared in the embodiments of the present invention have no toxic side effects on the organism with biological safety. Fig. 6 shows release and degradation of a selenium-doped black phosphorus prodrug prepared according to one embodiment of the present invention.

### Example 2

A method for preparing selenium-doped black phosphorus prodrug comprises the following steps.
(1) 500 mg of red phosphorus, 20 mg of tin, 10 mg of tin tetraiodide and 2.5 mg of selenium were sealed in a silicon glass vacuum tube.
(2) The silicon glass tube was placed horizontally in a heating furnace and heated at a heating rate of 4°C/min to 800°C and maintained for 3 hours, and then cooled at a cooling rate of 2°C/min to 550°C and maintained for 6 hours. Further, the glass tube was cooled at a cooling rate of 2°C/min to 100 °C and maintained for 8 hours, and then cooled to room temperature to obtain selenium-doped black phosphorus crystals deposited on the end of the silicon glass tube.
(3) The selenium-doped black phosphorus crystals were dispersed in the aqueous phase by liquid exfoliation to obtain suspended selenium-doped black phosphorus nanosheets, which were then coated with polyethylene glycol amine sequentially by ultrasonicating and stirring so as to obtain the selenium-doped black phosphorus prodrug, wherein the mass ratio of selenium-doped black phosphorus nanosheets to polyethylene glycol amine was 1:4. The ultrasonic treatment was performed at a frequency of 4500HZ for 2 hours, and the magnetic stirring was performed at a speed of 1000 rpm for 4 hours.

Fig. 7 shows a transmission electron microscope (SEM) image of a selenium-doped black phosphorus prodrug prepared in Example 2 of the present invention. The size of the selenium-doped black phosphorus prodrug is in a range of 50-100 nm.

### Example 3

A method for preparing selenium-doped black phosphorus prodrug comprises the following steps.
(1) 500 mg of red phosphorus, 20 mg of tin, 10 mg of tin tetraiodide and 5 mg of selenium were sealed in a silicon glass vacuum tube.
(2) The silicon glass tube was placed horizontally in a heating furnace and heated at a heating rate of 1°C/min to 800°C and maintained for 4 hours, and then cooled at a cooling rate of 1°C/min to 500°C and maintained for 8 hours. Further, the glass tube was cooled at a cooling rate of 1°C/min to 150 °C and maintained for 7 hours, and then cooled to room temperature to obtain selenium-doped black phosphorus crystals deposited on the end of the silicon glass tube.
(3) The selenium-doped black phosphorus crystals were dispersed in the aqueous phase by liquid exfoliation to obtain suspended selenium-doped black phosphorus nanosheets, which were then coated with polyethylene glycol amine sequentially by ultrasonicating and stirring so as to obtain the selenium-doped black phosphorus prodrug, wherein the mass ratio of selenium-doped black phosphorus nanosheets to polyethylene glycol amine was 1:2. The ultrasonic treatment was performed at a frequency of 3500HZ for 2 hours, and the magnetic stirring was performed at a speed of 800 rpm for 4 hours.

### Example 4

A method for preparing selenium-doped black phosphorus prodrug comprises the following steps.
(1) 500 mg of red phosphorus, 20 mg of tin, 10 mg of tin tetraiodide and 10 mg of selenium were sealed in a silicon glass vacuum tube.
(2) The silicon glass tube was placed horizontally in a heating furnace and heated at a heating rate of 5°C/min to 850°C and maintained for 4 hours, and then cooled at a cooling rate of 3°C/min to 550°C and maintained for 6 hours. Further, the glass tube was cooled at a cooling rate of 3°C/min to 150 °C and maintained for 10 hours, and then cooled to room temperature to obtain selenium-doped black phosphorus crystals deposited on the end of the silicon glass tube.
(3) The selenium-doped black phosphorus crystals were dispersed in the aqueous phase by liquid exfoliation to obtain suspended selenium-doped black phosphorus nanosheets, which were then coated with polyethylene glycol amine sequentially by ultrasonicating and stirring so as to obtain the selenium-doped black phosphorus prodrug, wherein the mass ratio of selenium-doped black phosphorus nanosheets to polyethylene glycol amine was 1:2. The ultrasonic treatment was performed at a frequency of 3000HZ for 2 hours, and the magnetic stirring was performed at a speed of 1200 rpm for 3 hours.

It should be noted that the foregoing descriptions are merely specific embodiments of the present invention, but are not intended to limit the protection scope of the present invention. Any variation or replacement readily figured out by a person skilled in the art within the technical scope disclosed in the present invention shall fall within the protection scope of the present invention. Therefore, the protection scope of the present invention shall be subject to the protection scope of the claims.

## Claims

1. A selenium-doped black phosphorus prodrug comprising:
selenium-doped black phosphorus nanosheets, and
polyethylene glycol amine coated on the surface of the selenium-doped black phosphorus nanosheets,
wherein the selenium-doped black phosphorus nanosheets comprises black phosphorus nanosheets and selenium doped in the black phosphorus nanosheets, and
wherein the selenium replaces a part of phosphorus atoms in the black phosphorus crystal lattice.

2. The selenium-doped black phosphorus prodrug of claim 1, wherein the selenium is doped in the selenium-doped black phosphorus nanosheets at a mass concentration of 0.1-5%.

3. The selenium-doped black phosphorus prodrug of claim 2, wherein the selenium is doped in the selenium-doped black phosphorus nanosheets at a mass concentration of 2-5%.

4. The selenium-doped black phosphorus prodrug of claim 2, wherein the selenium is doped in the selenium-doped black phosphorus nanosheets at a mass concentration of 1-4%.

5. The selenium-doped black phosphorus prodrug of claim 1, wherein length and width dimensions of the selenium-doped black phosphorus nanosheets are in a range of 50nm-200nm.

6. The selenium-doped black phosphorus prodrug of claim 5, wherein length and width dimensions of the selenium-doped black phosphorus nanosheets are in a range of 100nm-200nm.

7. The selenium-doped black phosphorus prodrug of claim 1, wherein thickness of the selenium-doped black phosphorus nanosheets is in a range of 1 nm-5nm.

8. The selenium-doped black phosphorus prodrug of claim 1, wherein mass ratio of the selenium-doped black phosphorus nanosheets to the polyethylene glycol amine is in a range of 1: (0.2-10).

9. The selenium-doped black phosphorus prodrug of claim 1, wherein the polyethylene glycol amine is adsorbed on the surface of the selenium-doped black phosphorus nanosheets due to electrostatic force, and wherein weight average molecular weight of polyethylene glycol amine is in a range of 2000-30000.

10. The selenium-doped black phosphorus prodrug of claim 1, wherein the polyethylene glycol amine comprises at least one compound selected from the group consisting of methyl polyethylene glycol amine, methoxy polyethylene glycol amine and polyethylene glycol diamine.

11. A method for preparing a selenium-doped black phosphorus prodrug, comprising:
sealing red phosphorus, tin, tin tetraiodide and selenium in a silicon glass vacuum tube at a mass ratio of (200-500): (10-20): (5-10): (0.5-10);
placing the silicon glass vacuum tube horizontally in a heating furnace, followed by heating the silicon glass vacuum to 700-800°C and maintaining for 1-5 hours, and then cooling to 450-550 °C and maintaining for 5-9 hours, and then further cooling to 100-200 °C and maintaining for 6-10 hours, followed by cooling to room temperature to obtain selenium-doped black phosphorus crystals in the silicon glass vacuum tube;
dispersing the selenium-doped black phosphorus crystals into an aqueous phase by liquid exfoliation to obtain suspended selenium-doped black phosphorus nanosheets;
coating the selenium-doped black phosphorus nanosheets with polyethylene glycol amine while ultrasonicating and stirring to obtain a selenium-doped black phosphorus prodrug, which comprises:
selenium-doped black phosphorus nanosheets, and
polyethylene glycol amine coated on the surface of the selenium-doped black phosphorus nanosheets,
wherein the selenium-doped black phosphorus nanosheets comprises black phosphorus nanosheets and selenium doped in the black phosphorus nanosheets, and
wherein the selenium replaces a part of phosphorus atoms in the black phosphorus crystal lattice.

12. The method for preparing a selenium-doped black phosphorus prodrug of claim 11, wherein the step of heating is performed at a heating rate of 1-5°C/min.

13. The method for preparing a selenium-doped black phosphorus prodrug of claim 11, wherein the step of cooling to 450-550°C is performed at a cooling rate of 1-3°C/min.

14. The method for preparing a selenium-doped black phosphorus prodrug of claim 11, wherein the step of cooling to 100-200°C is performed at a cooling rate of 1-3°C/min.

15. The method for preparing a selenium-doped black phosphorus prodrug of claim 11, wherein mass ratio of the selenium-doped black phosphorus nanosheets to polyethylene glycol amine is in a range of 1: (0.2-10).

16. The method for preparing a selenium-doped black phosphorus prodrug of claim 11, wherein weight average molecular weight of polyethylene glycol amine is in a range of 2000-30000.

17. The method for preparing a selenium-doped black phosphorus prodrug of claim 11, wherein the polyethylene glycol amine comprises at least one compound selected from the group consisting of methyl polyethylene glycol amine, methoxy polyethylene glycol amine and polyethylene glycol diamine.

18. The method for preparing a selenium-doped black phosphorus prodrug of claim 11, wherein mass of the selenium is 0.1-5% of mass of the red phosphorus.

19. The method for preparing a selenium-doped black phosphorus prodrug of claim 11, wherein mass of the tin is 2-10% of mass of the red phosphorus.

20. The method for preparing a selenium-doped black phosphorus prodrug of claim 11, wherein the ultrasonic treatment uses an ultrasonic frequency of 3000-4500HZ and lasts for 0.5-2 hours, and the step of stirring is performed at a speed of 800rpm-1200rpm for 2-4 hours.
